# EUROPEAN PATENT APPLICATION

(11) **EP 0 578 456 A1**
(43) Date of publication of application: **12.01.1994**
(21) Application number: 93305226.8
(22) Date of filing: 02.07.1993
(51) Int. Cl.: A43D 1/02, A61B 5/103

(54) **Apparatus and techniques for manufacturing insoles**

(30) Priority: 05.07.1992 IL 102415
(71) Applicant: Amsellem, Lucien, Herzliya 46851 (IL)
(72) Inventor: Amsellem, Lucien, Herzliya 46851 (IL)
(74) Representative: Raynor, John

(57) **Abstract**

Apparatus and technique for producing an insole based on the characteristics of the foot under dynamic loading of the kind occurring during walking or other activities.

## Description

### FIELD OF THE INVENTION

The present invention relates to apparatus and techniques for manufacturing insoles and to insoles manufactured thereby.

### BACKGROUND OF THE INVENTION

Various types of insoles are known. These include insoles in standard configurations which are matched to standard foot configurations as well as custom made insoles. Traditionally custom made insoles have been made by producing a plastic cast of the foot under a static load and using the cast to mold an appropriately shaped insole.

More recently molded in place custom insoles have been produced using various techniques. Such insoles are also based on the response of the foot to static loading thereof.

### SUMMARY OF THE INVENTION

The present invention seeks to provide apparatus and a technique for producing an insole based on the characteristics of the foot under dynamic loading of the kind occurring during walking or other activities.

There is thus provided in accordance with a preferred embodiment of the present invention apparatus for producing an insole including apparatus for sensing local forces on various locations of a foot under dynamic loading conditions, apparatus for providing a visually sensible imagewise output of patterns of such forces and apparatus for producing an insole based on the visually sensible imagewise output.

Additionally in accordance with a preferred embodiment of the present invention, there is provided a technique for producing an insole including the steps of sensing local forces on various locations of a foot under dynamic loading conditions, providing an imagewise output of force patterns on the foot, and producing an insole in order to modify the force patterns indicated by the imagewise output to a known acceptable range of force patterns.

Further in accordance with a preferred embodiment of the present invention, there is provided an insole constructed using the apparatus and/or the technique described above.

Additionally in accordance with a preferred embodiment of the present invention, there is provided an insole having a three-dimensional configuration based on observed force patterns in feet under dynamic loading and desired corrections thereto.

Further in accordance with a preferred embodiment of the present invention, there is provided an insole having a three-dimensional configuration based on reflexological characteristics of the foot.

Additionally in accordance with a preferred embodiment of the invention there is provided a non-custom made insert which is based on observed force patterns in a multiplicity of feet under dynamic loading and desired corrections thereto.

### BRIEF DESCRIPTION OF THE DRAWINGS

The present invention will be understood and appreciated more fully from the following detailed description, taken in conjunction with the drawings in which:
Fig. 1 is a general block diagram illustration of apparatus for producing insoles in accordance with a preferred embodiment of the present invention;
Fig. 2 is a flowchart illustrating a technique of producing insoles in accordance with a preferred embodiment of the present invention;
Fig. 3 is an exploded view of a typical insole produced in accordance with a preferred embodiment of the present invention;
Figs. 4 and 5 are pictorial illustrations of a typical insole in use; and
Fig. 6 is a pictorial illustration of an insole constructed in accordance with a preferred embodiment of the present invention.

### DETAILED DESCRIPTION OF A PREFERRED EMBODIMENT

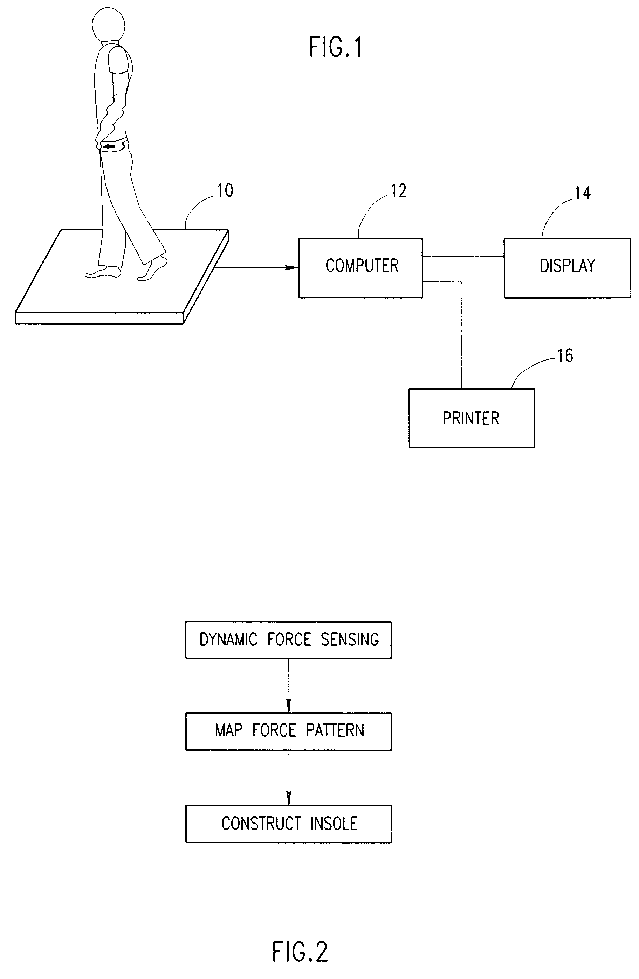

Reference is now made to Figs. 1 and 2 which illustrate apparatus and a technique for producing an insole in accordance with a preferred embodiment of the present invention. As seen in Fig. 1, a dynamic force sensing pad 10 is arranged to be walked upon by a person and to provide an output indication of the force pattern on the foot in response to dynamic loading thereof, as during walking.

The output indication from force sensing pad 10 is preferably supplied to a computer 12, such as a personal computer which is equipped with software enabling it to map the force pattern sensed by pad 10 in a visually sensible manner. Typical software for this purpose is commercially available from Ened Systems of Munich, Germany.

The visually sensible output from computer 12 may be provided on a display 14 and/or in hard copy format by means of a printer 16.

Using the output from computer 12 together with known information regarding desired force patterns in feet under dynamic loading, an operator selects a suitable three dimensional configuration of an insole. This may be done by custom forming a unitary insole member or alternatively combining a plurality of existing three dimensional elements in an appropriate spatial relationship.

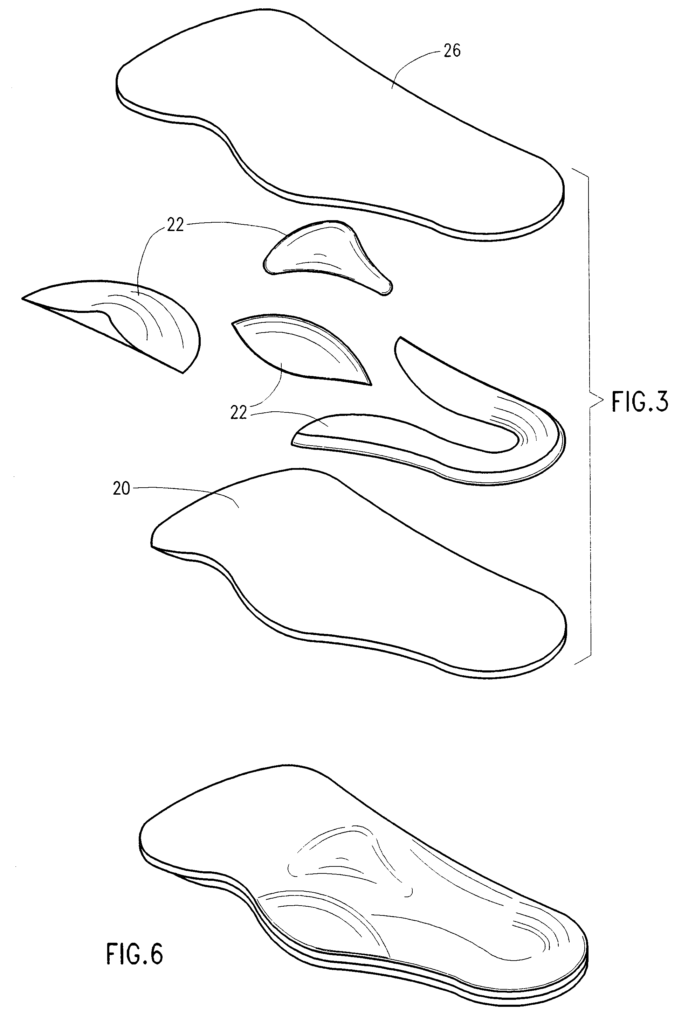

Once the insole configuration has been determined, it may be assembled from a plurality of layers as illustrated in Fig. 3, including preferably an impact absorbing bottom layer 20, such as Noene, which is manufactured by Universal Plast S.r.l. of Brescia, Italy, one or more three dimensional configuration determining elements 22, typically formed of foamed rubber or plastic, and a top layer 26, typically formed of leather.

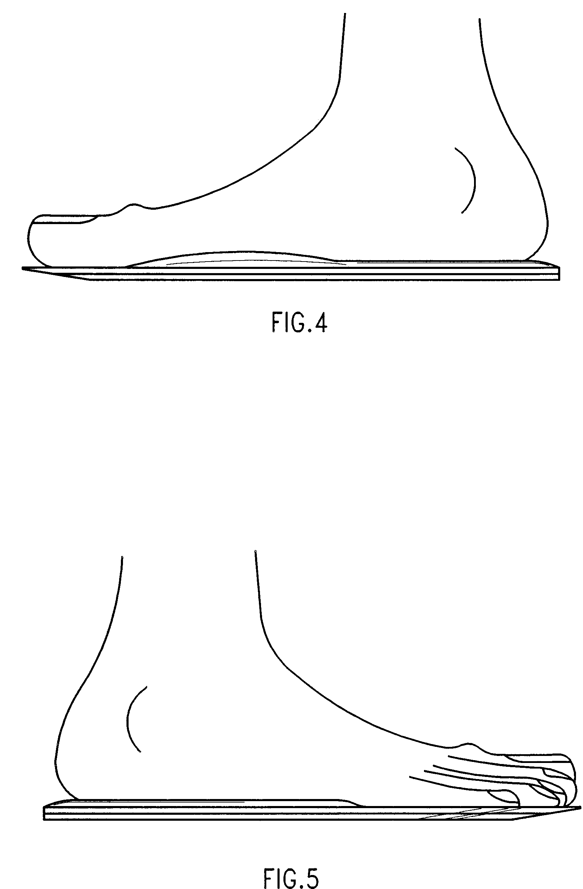

A typical insole is illustrated in Fig. 6 and is shown in use in Figs. 4 and 5. It is appreciated that non-custom made insoles can be made based on force diagrams produced using the apparatus and techniques described hereinabove.

It will be appreciated by persons skilled in the art that the present invention is not limited by what has been particularly shown and described hereinabove. Rather the scope of the present invention is defined only by the claims which follow:

## Claims

1. Apparatus for producing an insole including apparatus for sensing local forces on various locations of a foot under dynamic loading conditions, apparatus for providing a visually sensible imagewise output of patterns of such forces and apparatus for producing an insole based on the visually sensible image-wise output.

2. A technique for producing an insole including the steps of sensing local forces on various locations of a foot under dynamic loading conditions, providing an imagewise output of force patterns on the foot, and producing an insole in order to modify the force patterns indicated by the imagewise output to a known acceptable range of force patterns.

3. An insole constructed using the apparatus of claim 1.

4. An insole constructed using the technique of claim 2.

5. An insole having a three-dimensional configuration based on observed force patterns in feet under dynamic loading and desired corrections thereto.

6. An insole having a three-dimensional configuration based on reflexological characteristics of the foot.

7. A non-custom made insole having a three-dimensional configuration which is based on observed force patterns in a multiplicity of feet under dynamic loading and desired corrections thereto.

8. A non-custom made insole made in accordance with the technique of claim 2.

9. A non-custom made insole made using the apparatus of claim 1.

10. An insole according to any one of claims 3 to 6, which is non-custom made.
